# EUROPÄISCHE PATENTSCHRIFT

(11) **EP 1 585 716 B1**
(45) Veröffentlichungstag und Bekanntmachung des Hinweises auf die Patenterteilung: **21.11.2012**
(21) Anmeldenummer: 03785939.4
(22) Anmeldetag: 24.12.2003
(51) Int. Cl.: C07C 51/44, C07C 45/82, C07C 67/54, C07C 45/78, C07C 45/81

(54) **THERMISCHES TRENNVERFAHREN ZWISCHEN WENIGSTENS EINEM GASFÖRMIGEN UND WENIGSTENS EINEM FLÜSSIGEN STOFFSTROM, VON DENEN WENIGSTENS EINER( METH)ACRYLMONOMERE ENTHÄLT**
THERMAL SEPARATING METHOD FOR SEPARATING AT LEAST ONE GASEOUS MATERIAL FLOW FROM AT LEAST ONE LIQUID MATERIAL FLOW, AT LEAST ONE OF SAID FLOWS CONTAINING (METH)ACRYLIC MONOMERS
PROCEDE DE SEPARATION THERMIQUE ENTRE AU MOINS UN FLUX DE SUBSTANCE GAZEUSE ET AU MOINS UN FLUX DE SUBSTANCE LIQUIDE, DONT AU MOINS L'UN CONTIENT DES MONOMERES (METH)ACRYLIQUES

(30) Priorität: 10.01.2003 DE 10300816
(43) Veröffentlichungstag der Anmeldung: 19.10.2005
(73) Patentinhaber: BASF SE, 67056 Ludwigshafen (DE)
(72) Erfinder: THIEL, Joachim, 67435 Neustadt (DE); VANDENMERSCH, Hugues, 67157 Wachenheim (DE); SCHRÖDER, Jürgen, 67071 Ludwigshafen (DE); DAMS, Albrecht, 67157 Wachenheim (DE)
(86) Internationale Anmeldenummer: PCT/EP2003/014879
(87) Internationale Veröffentlichungsnummer: WO 2004/063138

(56) Entgegenhaltungen:
- WO-A-00/53561
- DE-A- 19 924 532

## Beschreibung

Vorliegende Erfindung betrifft ein thermisches Trennverfahren zwischen wenigstens einem gasförmigen und wenigstens einem flüssigen Stoffstrom, von denen wenigstens einer (Meth)acrylmonomere enthält, in einer trennwirksame Einbauten enthaltenden Trennkolonne, wobei es sich wenigstens bei einem Teil der trennwirksamen Einbauten um eine Abfolge von Siebböden handelt.

Die Schreibweise (Meth)acrylmonomere steht in dieser Schrift verkürzend für"Acrylmonomere und/oder Methacrylmonomere".

Der begriff Acrylmonomere steht in dieser Schrift verkürzend für "Acrolein, Acrylsäure und/oder Ester der Acrylsäure".

Der Begriff Methacrylmonomere steht in dieser Schrift verkürzend für "Methacrolein, methacrylsäure und/oder Ester der Methacrylsäure".

Im besonderen sollen die in dieser Schrift angesprochenen (Meth)acrylmonomeren die nachfolgenden (Meth)acrylsäureester umfassen: Hydroxyethylacrylat, Hydroxyethylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, isoButylmethacrylat, n-Butylmethacrylat, tert.-Butylacrylat, tert.-Butylmethacrylat, Ethylacrylat, Ethylmethacrylat, 2-Ethylhexylacrylat, 2-Ethylhexylmethacrylat, N,N-Dimethylaminoethylacrylat und N,N-Dimethylaminoethylmethacrylat.

(Meth)acrylmonomere sind wichtige Ausgangsverbindungen zur Herstellung von Polymerisaten, die z.B. als Klebstoff Verwendung finden.

(Meth)acrolein und (Meth)arylsäure wird großtechnisch überwiegend durch katalytische Gasphasenoxidation geeigneter C3-/C4-Vorläuferverbindungen (oder von Vorläuferverbindungen derselben), insbesondere von Propen und Propan im Fall von Acrolein und Acrylsäure bzw. iso-Buten und iso-Butan im Fall der Methacrylsäure und des Methacroleins, hergestellt. Neben Propen, Propan, iso-Buten und iso-Butan eignen sich als Ausgangsstoffe jedoch auch andere 3 bzw. 4 Kohlenstoffatome enthaltende Verbindungen wie iso-Butanol, n-Propanol oder Vorläuferverbindungen derselben wie z.B. der Methylether von iso-Butanol. (Meth)acrylsäure kann auch aus (Meth)acrolein erzeugt werden.

Dabei wird normalerweise ein Produktgasgemisch erhalten, aus dem die (Meth)acrylsäure bzw. das (Meth)acrolein abgetrennt werden muss.

Ester der (Meth)acrylsäure sind z.B. durch direkte Umsetzung von Methylacrylsäure und/oder (Meth)acrolein mit den entsprechenden Alkoholen erhältlich. Allerdings fallen auch in diesem Fall zunächst Produktgemische an, aus denen die (Meth)acrylsäureester abgetrennt werden müssen.

Für vorstehende Abtrennungen werden häufig Trennverfahren angewendet, die in trennwirksame Einbauten enthaltenden Trennkolonnen durchgeführt werden. In diesen Trennkolonnen werden vielfach gasförmige (aufsteigend) und flüssige (absteigend) Stoffströme im Gegenstrom geführt, wobei infolge des zwischen den Stoffströmen bestehenden Ungleichgewichts ein Wärme- und Stoffaustausch stattfindet, der letztlich die in der Trennkolonne gewünschte Auftrennung bedingt. In dieser Schrift sollen solche Trennverfahren als thermische Trennverfahren bezeichnet werden.

Beispiel für und damit Element des in dieser Schrift verwendeten Begriffs "thermische Trennverfahren" sind die fraktionierende Kondensation (vgl. DE-A 19924532) und/oder die Rektifikation (aufsteigende Dampfphase wird im Gegenstrom zu absteigender Flüssigphase geführt; die Trennwirkung beruht darauf, dass die Dampfzusammensetzung im Gleichgewicht anders als die Flüssigzusammensetzung ist), die Absorption (wenigstens ein aufsteigendes Gas wird zu wenigstens einer absteigenden Flüssigkeit im Gegenstrom geführt; die Trennwirkung beruht auf der unterschiedlichen Löslichkeit der Gasbestandteile in der Flüssigkeit), die Strippung (wie die Absorption; die Flüssigphase ist jedoch mit einer Komponente beladen, die vom Strippgas aufgenommen wird) und die Desorption (der Umkehrprozess zur Absorption; das in der Flüssigphase gelöste Gas wird durch Partialdruckerniedrigung abgetrennt).

Beispielweise kann die Abtrennung von (Meth)acrylsäure bzw. (Meth)acrolein aus dem Produktgasgemisch der katalytischen Gasphasenoxidation so durchgeführt werden, dass die (Meth)acrylsäure bzw. das (Meth)acrolein durch Absorption in ein Lösungsmittel (z.B. Wasser oder ein organisches Lösungsmittel) oder durch fraktionierende Kondensation des Produktgasgemisches zunächst grundabgetrennt und das dabei anfallende Kondensat bzw. Absorbat nachfolgend rektifikativ (in der Regel in mehreren Stufen) unter Erhalt von mehr oder weniger reiner (Meth)acrylsäure bzw. (Meth)acrolein aufgetrennt wird (vgl. z.B. EP-A 717019, EP-A 1125912, EP-A 982289, EP-A 982287, DE-A 19606877, DE-A 1011527, DE-A 10224341 und DE-A 10218419).

Die vorstehend angesprochene fraktionierende Kondensation unterscheidet sich von der herkömmlichen Rektifikation im wesentlichen dadurch, dass das aufzutrennende Gemisch der Trennkolonne gasförmig (d.h. vollständig in die Dampfform überführt) zugeführt wird.

Die bereits angesprochenen, (Meth)acrylmonomere enthaltenden, gasförmigen bzw. flüssigen Gemische können die (Meth)acrylmonomere sowohl in mehr oder weniger reiner Form als auch in Verdünnung (z.B. mit Lösungsmittel oder mit Verdünnungsgasen) befindlich enthalten. Das Lösungsmittel kann dabei sowohl wässrig als auch ein organisches Lösungsmittel sein, wobei die spezifische Art des organischen Lösungsmittels im wesentlichen unbeachtlich ist. Das Verdünnungsgas kann z.B. Stickstoff, Kohlenoxid (CO, CO₂), Sauerstoff, Kohlenwasserstoff oder ein Gemisch aus diesen Gasen sein.

Das heißt, z.B. auf dem Weg der Gewinnung von (Meth)acrylmonomeren werden auf unterschiedlichste Art und Weise thermische Trennverfahren auf gasförmige und/oder flüssige Stoffgemische angewendet, deren Gehalt an (Meth)acrylmonomeren ≥ 2: 2 Gew-%, oder ≥ 10 Gew.-%, oder ≥ 20 Gew.-%, oder ≥ 40 Gew.-%, oder ≥ 60 Gew.-%, oder ≥ 80 Gew.-%, oder ≥ 90 Gew.-%, oder ≥ 95 Gew.-%, oder ≥ 99 Gew.-% betragen kann.

Die Anreicherung der (Meth)acrylmonomere kann dabei sowohl am Kopf als auch im Sumpf der Trennkolonne erfolgen. Selbstredend können aber auch im oberen, unteren oder mittleren Teil der Trennkolonne (Meth)acrylmonomere angereichert enthaltende Fraktionen entnommen werden.

Die in den Trennkolonnen enthaltenen trennwirksamen Einbauten verfolgen bei den thermischen Trennverfahren den Zweck, die Oberfläche für den die Auftrennung in der Trennkolonne bewirkenden Wärme- und Stoffaustausch zu erhöhen.

Als solche Einbauten kommen z.B. Packungen, Füllkörper und/oder Stoffaustauschböden in Betracht.

Besonders häufig werden als Trennkolonnen solche verwendet, die wenigstens als einen Teil der trennwirksamen Einbauten eine Abfolge von Stoffaustauschböden enthalten.

Stoffaustauschböden verfolgen den Zweck, in der Trennkolonne in Form von Flüssigkeitsschichten Orte mit geschlossenen flüssigen Phasen zur Verfügung zu stellen. Die Oberfläche des in der Flüssigkeitsschicht aufsteigenden und sich dabei in der geschlossenen flüssigen Phase verteilenden Dampf- bzw. Gasstromes ist dann die maßgebende Austauschfläche.

Ein Klassiker unter den Stoffaustauschböden ist der Siebboden. Darunter sollen in dieser Schrift Platten verstanden werden, die als Durchtrittsstellen für die aufsteigende Gas- bzw. Dampfphase (die begriffe "gasförmig" und "dampfförmig" werden in dieser Schrift synonym verwendet) einfache Löcher und/oder Schlitze aufweisen.

Die Siebböden werden dabei in zwei Gruppen differenziert, nämlich in solche mit Flüssigkeitszwangsführung und solche ohne Flüssigkeitszwangsführung.

Die Flüssigkeitszwangsführung wird dabei dadurch erzielt, dass die Siebböden neben den Durchtrittsstellen für die aufsteigende Gas- bzw. Dampfphase wenigstens einen Ablaufschacht aufweisen (Ablauf), durch den die Flüssigkeit unabhängig vom Strömungsweg des Dampfes vom höher gelegenen Boden auf den nächsten tiefer gelegenen Boden fließt (Zulauf). Die Flüssigkeit fließt im Querstrom über den Boden vom wenigstens einen Zulauf zum wenigstens einen Ablauf, wobei das Zulauf- und Ablaufrohr den Flüssigkeitsverschluss und die gewünschte Flüssigkeitshöhe auf dem Boden garantieren. Häufig (insbesondere bei geringen Kolonnendurchmessern) sind die Siebböden mit Flüssigkeitszwangsführung einflutig gestaltet. D.h., Zulauf und Ablauf sind auf gegenüberliegenden Seiten des Bodens angeordnet. Sie können aber auch zweiflutig (oder auch mehr als zweiflutig) gestaltet sein. In diesem Fall kann der-Zulauf z.B. in der Mitte und je ein Ablauf auf gegenüberliegenden Seiten des Stoffaustauschbodens angeordnet sein. Nachfolgend sollen solche Siebböden, als Zwangssiebböden bezeichnet werden. Bei ihnen wird ein die Trennwirkung minderndes Durchregnen der Flüssigkeit nicht wie beim Glockenboden durch Kamine verhindert, in die die Durchtrittsöffnungen fortführen, sondern es bedarf dazu einer minimalen Dampfbelastung. Der Dampf tritt aufsteigend durch die Durchtrittsöffnungen und durchperlt die vom Ablaufrohr gehaltene Flüssigkeitsschicht.

Von den Zwangssiebböden unterscheiden sich die Dual-Flow- oder auch Regensiebböden dadurch, dass sie kein Ablaufsegment enthalten. Durch die Abwesenheit von Ablaufsegmenten (Ablaufschächten) treten bei den Regensiebböden das aufsteigende Gas und die in der Trennkolonne absteigende Flüssigkeit durch die gleichen Durchtrittsstellen des Bodens. Auch beim Regensiebboden bedarf es wie beim Zwangssiebboden einer minimalen Dampfbelastung, um eine angemessene Trennwirkung zu erzielen. Wird sie signifikant unterschritten, bewegen sich aufsteigendes Gas und absteigender Rücklauf im wesentlichen ohne Austausch aneinander vorbei und der Boden läuft Gefahr, trocken zu laufen. D.h., auch beim Regensiebboden muss eine untere Grenzgeschwindigkeit vorhanden sein, damit auf dem Boden eine gewisse Flüssigkeitsschicht gehalten wird, um ein Arbeiten des Bodens zu ermöglichen. Im normalen Arbeitsbereich regnet die Flüssigkeit bei Regensiebböden durch die Durchtrittsöffnungen von Boden zu Boden und zwischen den Böden wird die geschlossene Gasphase von einer zerteilten Flüssigkeitsphase durchsetzt. Die auf dem Regensiebboden auftreffenden Tropfen werden teilweise versprüht.

Während ein Vorteil von Siebböden gegenüber Glockenböden in ihrer einfacheren Bauweise begründet ist, besteht einer ihrer Nachteile darin, dass bei ihnen durch die stets nach oben gerichtete Strömungsrichtung des Dampfes die Neigung zum Mitreißen kleiner Flüssigkeitstropfen erhöht ist. Infolge des Mitreißens von Flüssigkeit durch den aufsteigenden Dampf von einem tiefer gelegenen Siebboden zum nächst höher gelegenen Siebboden wird die Gegenstromführung von Gas- und Flüssigphase in der Trennkolonne beeinträchtigt. Es kommt dadurch zu einer Rückvermischung der Flüssigkeit über die Siebböden, wodurch das den Stoffaustausch treibende Konzentrationsgefälle und somit der Stoffübergang zwischen den Phasen und damit letztlich die Trennwirkung gemindert wird.

Nachfolgend soll der Gewichtsanteil der gesamten in einer in Betrieb befindlichen Trennkolonne einem Siebboden zugeführten Flüssigkeitsmenge, die durch das aufsteigende Gas zum nächst höher gelegenen Siebboden mitgerissen wird, als Mitrissanteil (in Gew.-%) dieses Siebbodens bezeichnet werden.

Prinzipiell lässt sich der Mitrissanteil eines Siebbodens in einer in Betrieb (Durchführung eines thermischen Trennverfahrens) befindlichen Trennkolonne experimentell bestimmen. Beispielsweise kann man bei einer Rektifikation in einer Trennkolonne, die als trennwirksame Einbauten ausschließlich Regensiebböden in äquidistantem Abstand d enthält, im Abstand d oberhalb des obersten Regensiebbodens einen Kaminboden (z.B. einen solchen gemäß DE-A 10159825) anbringen. Die durch den Kaminboden aufsteigende Dampfphase wird aus der Kolonne herausgeführt und in einem Kondensator kondensiert. Ein Teil des Kondensats wird als Reinprodukt weggeführt und der verbleibende Teil als Rücklaufflüssigkeit zwischen dem Kaminboden und dem obersten Siebboden in die Trennkolonne rückgeführt. Sie bildet die erste Teilmenge der Gesamtmenge der dem obersten Siebboden zugeführten Flüssigkeitsmenge. Beim Durchtritt der Dampfphase durch die Kamine des Kaminbodens scheiden sich in der Dampfphase befindliche mitgerissene Flüssigkeitströpfchen auf dem Kaminboden (Fangboden) ab. Die sich dadurch auf dem Kaminboden ausbildende Flüssigphase wird diesem kontinuierlich entnommen, in ihrer Menge bestimmt und als die zweite Teilmenge der Rücklaufflüssigkeit zugeführt. Aus Gesamtmenge und zweiter Teilmenge lässt sich der Mitrissanteil des obersten Siebbodens bestimmen. Dabei zeigt sich, dass der Mitrissanteil im wesentlichen unabhängig von der genauen Stelle ist, an der die Rücklaufflüssigkeit zwischen Kaminboden und oberstem Regensiebboden in die Trennkolonne rückgeführt wird.

Für andere Bodenlagen kann der Mitrissanteil in ähnlicher Weise experimentell ermittelt werden. Dabei zeigt sich, dass sich in einer reinen Siebbodenkolonne mit identischen, äquidistant angeordneten Siebböden von nicht zu großer Gesamtzahl der Mitrissanteil innerhalb der Trennkolonne von oben nach unten bei einem thermischen Trennverfahren nur geringfügig ändert.

Da im unteren Teil einer Trennkolonne der Druck erhöht ist, nimmt die Massendichte in einer Trennkolonne bei einem thermischen Trennverfahren von oben nach unten zu, weshalb im unteren Kolonnenteil ein bestimmter Massenstrom bereits bei einer vergleichsweise etwas geringeren Gasgeschwindigkeit erreicht wird, woraus letztlich ein etwas kleinerer Mitrissanteil resultiert.

Inzwischen ist es auch möglich, die Mitrissanteile von Siebböden aus hydrodynamischen Messgrößen sowie der Bodenbeschaffenheit (z.B. Lochdurchmesser, Lochabstand, Öffnungsverhältnis, Bodenabstand etc.) zu errechnen.

Ein anderes Problemfeld bei der Durchführung thermischer Trennverfahren zwischen wenigstens einem gasförmigen und wenigstens einem flüssigen Stoffstrom, von denen wenigstens einer (Meth)acrylmonomere enthält, in einer Siebböden enthaltende Trennkolonne besteht darin, dass (Meth)acrylmonomere bezüglich ihrer radikalischen Polymerisation sehr reaktiv sind und zu unerwünschter Polymerisation neigen. Eine solche unerwünschte Polymerisation ist insbesondere auf der Unterseite von Siebböden kritisch, da diese bei üblichem Betrieb eines thermischen Trennverfahrens in einer Siebböden enthaltenden Trennkolonne weitgehend trocken sind. Sich auf der Unterseite im Normalbetrieb ausbildendes Polymerisat kann daher weitgehend ungestört aufwachsen und die Siebböden einer Trennkolonne letztendlich bereits nach kurzer Betriebsdauer verstopfen und den weiteren Betrieb einer Trennkolonne unmöglich machen.

Eine Verbesserung kann dadurch erzielt werden, dass der Lehre der DE-A 2027655 folgend Siebböden mit speziell ausgeformten Löchern verwendet werden, deren Lochform gewährleistet, dass flüssiger Rücklauf nach Durchtritt durch die Löcher die Bodenunterseite kontinuierlich befeuchtet. Sich auf der Siebbodenunterseite ausbildende Polymerisationskeime werden so kontinuierlich weggeschwemmt und in den Kolonnensumpf transportiert, was ein Polymerisataufwachsen auf der Siebbodenunterseite mindert. Eine weitere Verbesserung wird dann erzielt, wenn die Trennkolonne polymerisationsinhibiert betrieben wird. D.h., der in der Trennkolonne absteigenden Flüssigphase werden, wie bei thermischen Trennverfahren unter Beteiligung von (Meth)acrylmonomeren üblich, Polymerisationsinhibitoren (z.B. phenolische Verbindungen, Aminoverbindungen, Nitroverbindungen, Phosphorverbindungen, Schwefelverbindungen, N-Oxylverbindungen und/oder Schwermetallsalze) zugesetzt. Eine Befeuchtung der Bodenunterseite gemäß der DE-A 2027655 führt in diesem Fall automatisch auch zu einer Polymerisationsinhibierung der Siebbodenunterseite.

Nachteilig an der Verfahrensweise der DE-A 2027655 ist jedoch, dass die spezielle Ausformung der Löcher den wesentlichen Vorteil der Siebböden, nämlich ihre einfache Herstellbarkeit, aufgibt.

Die EP-A 937488 und die EP-A 1044957 beschreiben Verfahren der Rektifikation von (Meth)acrylmonomere enthaltenden Stoffgemischen, bei denen die innere Oberfläche der Rektifikationkolonne, einschließlich der Siebbodenunterseite, über Düsen mit polymerisationsinhibiertem Rücklauf besprüht wird.

Nachteilig an dieser Verfahrensweise ist, dass sie einen zusätzlichen apparativen Aufwand bedeutet.

Die EP-A 1029573 empfiehlt zur Polymerisationsminderung bei Verfahren der Rektifikation von (Meth)acrylmonomere enthaltenden Stoffgemischen die Anwendung von Dual-Flow-Böden, deren Lochdurchmesser, Lochabstände, Bodendicken, Öffnungsverhältnisse, Lochform, Bodenabstand und Flüssigkeitsbelastung in vergleichsweise eng definierten Bereichen liegen. Doch vermögen auch diese Maßnahmen zur Minderung einer unerwünschten radikalischen Polymerisation von (Meth)acrylmonomeren nicht voll zu befriedigen.

Die Aufgabe der vorliegenden Erfindung bestand daher darin, unter dem Aspekt einer Minderung von unerwünschter Polymerisatbildung ein verbessertes thermisches Trennverfahren zwischen wenigstens einem gasförmigen und wenigstens einem flüssigen Stoffstrom, von denen wenigstens einer (Meth)acrylmonomere enthält, in einer trennwirksame Einbauten enthaltenden Trennkolonne, wobei es sich wenigstens bei einem Teil der trennwirksamen Einbauten um eine Abfolge von Siebböden handelt, zur Verfügung zu stellen.

Demgemäss wurde ein thermisches Trennverfahren zwischen wenigstens einem gasförmigen und wenigstens einem flüssigen Stoffstrom, von denen wenigstens einer (Meth)acrylmonomere enthält, in einer trennwirksame Einbauten enthaltenden Trennkolonne, wobei es sich wenigstens bei einem Teil der trennwirksamen Einbauten um eine Abfolge von Siebböden handelt, gefunden, das dadurch gekennzeichnet ist, dass die Stoffströme so gewählt werden, dass dabei wenigstens ein Teil der Siebböden oberhalb eines Mitrissanteils von 10 Gew.-% betrieben wird.

Der Gegenstand der WO 00/53561 unterscheidet sich vom Gegenstand der vorliegenden Anmeldung darin, dass das Merkmal "die Stoffströme so gewählt sind, dass dabei wenigstens ein Teil der Siebböden oberhalb eines Mitrissanteils von 10 Gew.-% betrieben wird" nicht offenbart ist.

Erfindungsgemäß überrascht, dass im Gegensatz zur gängigen Lehre (z.B. Johann Stichlmair in Grundlagen der Dimensionierung des Gas-/Flüssigkeit-Kontaktapparates, Bodenkolonne, Verlag Chemie, Weinheim, 1978, S. 131) die Trennwirkung von Siebböden auch bei Mitrissanteilen von bis zu 30 Gew.-% kaum spürbar gemindert wird.

Ein erhöhter Mitrissanteil führt jedoch automatisch zu einer erhöhten Befeuchtung der Bodenunterseiten in einer innerhalb einer Trennkolonne befindlichen Siebbodenabfolge und mindert dadurch in ähnlicher, aber einfacherer Art und Weise wie in der DE-A 2027655 beschrieben unerwünschte Polymerisatbildung. Dies gilt insbesondere dann, wenn die beim erfindungsgemäßen thermischen Trennverfahren in der Trennkolonne absteigende Flüssigphase in an sich bekannter Weise Polymerisationsinhibitoren zugesetzt enthält. Als ein solcher Polymerisationsinhibitor kann auch ein molekularen Sauerstoff enthaltendes Gas mit dem aufsteigenden Dampf durch die Trennkolonne geführt oder an unterschiedlichen Stellen in der Trennkolonne eingedüst werden. In einfachster Weise kann ein solches molekularen Sauerstoff enthaltendes Gas Luft sein (vgl. z.B. DE-A 10248606, DE-A 10238142 und DE-A 10217121).

D.h., der Mitrissanteil wenigstens eines Teils der Siebböden kann bei Durchführung des erfindungsgemäßen Verfahrens ohne nennenswerte Minderung der Trennwirkung > 10 bis 30 Gew.-%, oder 11 bis 30 Gew.-%, oder 12 bis 30 Gew.-%, oder 13 bis 30 Gew.-%, oder 14 bis 30 Gew.-%, oder 15 bis 30 Gew.-% betragen. Als Obergrenze der genannten Bereiche kommen anstelle der 30 Gew.-% auch 28 Gew.-%, oder 25 Gew.-%, oder 20 Gew.-% in Betracht.

Erfindungsgemäß bevorzugt wird das erfindungsgemäße thermische Trennverfahren so durchgeführt, dass der Mitrissanteil wenigstens der Hälfte und besonders bevorzugt wenigstens 75 % oder aller Siebböden in den vorgenannten Bereichen liegt. Insbesondere sollten diejenigen Siebböden in den vorgenannten Bereichen liegen, auf denen der Gehalt an (Meth)acrylmonomeren besonders hoch ist.

Dies gilt insbesondere dann, wenn die Trennkolonne als trennwirksame Einbauten ausschließlich Siebböden enthält (Zwangssiebböden und/oder Regensiebböden). Insbesondere gilt es dann, wenn die Abfolge der Siebböden beim erfindungsgemäßen Verfahren äquidistant ist.

Beobachtet man bei der Ausübung des erfindungsgemäßen Verfahrens im Vergleich zum Normalbetrieb der Siebböden gemäß der Lehre des Standes der Technik eine Minderung der Trennwirkung der in der Trennkolonne enthaltenen Abfolge von Siebböden, so kann dies dadurch ausgeglichen werden, dass man die Anzahl der Siebböden bei gleichbleibendem Abstand (d.h., die Kolonnenhöhe) erhöht.

Anwendungstechnisch zweckmäßig sollte der Siebbodenabstand innerhalb der Siebbodenabfolge sich im Bereich von 300 mm bis 900 mm bewegen. Erfindungsgemäß bevorzugt beträgt beim erfindungsgemäßen Verfahren der Siebbodenabstand innerhalb der Siebbodenabfolge 300 bis 500 mm. Im Regelfall sollten der Siebbodenabstand 250 mm nicht unterschreiten.

Mittels der Maßnahme der Erhöhung der Anzahl der Siebböden ist es beim erfindungsgemäßen Verfahren möglich, den Mitrissanteil der Siebböden auf Werte von bis zu 70 Gew.-% zu steigern, ohne die Trennwirkung nennenswert zu beeinträchtigen. D.h., die Obergrenze des Mitrissanteils wenigstens eines Teils der Siebböden kann bei Durchführung des erfindungsgemäßen Verfahrens für die bereits genannten Bereiche anstelle der 30 Gew.-% auch 35 Gew.-%, oder 40 Gew.-% oder 50 Gew.-%, oder 60 Gew.-%, oder 70 Gew.-% betragen. Selbstredend können beim erfindungsgemäßen Verfahren auch die Mitrissanteile aller Siebböden in diesem erweiterten Mitrissanteil liegen.

Als (Meth)acrylmonomere kommen für das erfindungsgemäße Verfahren alle diejenigen in Betracht, die eingangs dieser Schrift genannt worden sind. Es kann eine fraktionierende Kondensation, oder eine Rektifikation, oder eine Absorption, oder eine Strippung, oder eine Desorption sein.

Insbesondere kann das erfindungsgemäße Verfahren auf alle thermischen Verfahren der Abtrennung von (Meth)acrylmonomeren aus den eingangs dieser Schrift erwähnten Stoffgemischen angewendet werden.

Dabei kann der Gehalt der gasförmigen und/oder flüssigen Stoffgemische an (Meth)acrylmonomeren ≥ 2 Gew.-%, oder ≥10 Gew.-%, oder ≥ 20 Gew.-%, oder ≥ 40 Gew.-%, oder ≥ 60 Gew.-%, oder ≥ 80 Gew.-%, oder ≥ 90 Gew.-%, oder ≥ 95 Gew.-%, oder ≥ 99 Gew.-% betragen.

Die Siebböden selbst können beim erfindungsgemäßen Verfahren wie in der DE-A 2027655, der DE-A 10156988, der DE-A 10230219, der EP-A 1029573 oder in Grundlagen der Dimensionierung von Kolonnenböden, Technische Fortschrittsberichte, Band 61, K. Hoppe, M. Mittelstrass, Verlag Theodor Steinkopff, Dresden 1967 beschrieben gestaltet werden. Dabei können die Durchtrittsöffnungen kreisförmig, elliptisch oder vieleckig gestaltet sein. Sie können auch jedwede andere Form (z.B. schlitzförmig) aufweisen. Erfindungsgemäß bevorzugt sind sie kreisförmig und in strenger Dreiecksteilung angeordnet. Beispielsweise kann der Lochdurchmesser der Siebböden (insbesondere im Fall von Dual-Flow-Böden) 5 bis 50 mm, bevorzugt 10 bis 25 mm betragen. Der Abstand zweier nächstliegender Lochmittelpunkte beträgt zweckmäßig das1,5-bis 3-fache, bevorzugt das 2- bis 2,8-fache des Lochdurchmessers, welcher über den einzelnen Siebboden bevorzugt einheitlich dimensioniert ist.

Das Öffnungsverhältnis (Verhältnis der Gesamtfläche aller Durchtrittsöffnungen des Siebbodens zur Gesamtfläche des Siebbodens multipliziert mit 100 und in %) beträgt bei erfindungsgemäß einzusetzenden Siebböden zweckmäßig 8 bis 30 % und häufig 12 bis 20 %. Die Bodendicke liegt günstig bei 1 bis 8 mm.

Erfindungsgemäße Verfahren sind z.B. Rektifikationen oder fraktionierende Kordensationen, die in Trennkolonnen durchgeführt werden, die als trennwirksame Einbauten ausschließlich Böden enthalten, von deren Anzahl wenigstens zwei, bevorzugt mehr als zwei (bevorzugt ≥ 10 %, oder ≥ 20 %, oder ≥ 30 %, oder ≥ 40 %, oder ≥ 50 %, oder ≥ 60 %, oder ≥ 75 %) und besonders bevorzugt alle Siebböden, mit besonderem Vorteil Regensiebböden mit kreisförmigen Durchtrittsöffnungen sind.

Die übrigen Böden können z.B. hydraulisch abgedichtete Querstromböden (z.B. Thormann-Böden oder Glockenböden) und/oder Ventilböden sein.

Der Gasbelastungsfaktor F der erfindungsgemäß anzuwendenden Abfolge von Siebböden liegt in der Praxis vielfach im Bereich von 1 bis 3 Pa^{0,5}, häufig im Bereich von 1,5 bis 2,5 Pa^{0,5}. Die Flüssigkeitsgeschwindigkeit liegt gleichzeitig oft im Bereich von 1 bis 50 m/h oder im Bereich von 2 bis 10 m/h.

Wie bereits erwähnt, wird das erfindungsgemäße Verfahren, insbesondere im Fall einer Rektifikation oder Absorption, normalerweise polymerisationsinhibiert betrieben. In der Regel werden dazu die Polymerisationsinhibitoren am Kopf der Trennkolonne in die in der Trennkolonne absteigende Flüssigphase (z.B. die Rücklaufflüssigkeit oder das Absorptionsmittel) gegeben. Als in typischer Weise erfindungsgemäß einsetzbare Polymerisationsinhibitoren seien Phenothiazin, Hydrochinon und der Monomethylether des Hydrochinons genannt. Als weitere Stabilisierungsmaßnahme kann zusätzlich, wie ebenfalls bereits beschrieben, ein molekularen Sauerstoff enthaltendes Gas, z. B. Luft, durch die Trennkolonne geführt werden. In günstigen Fällen kann auch ausschließlich mit Luft polymerisationsinhibiert werden.

Vorteilhaft werden bei erfindungsgemäß eingesetzten Regensiebböden Quervermischungen und großflächige Wellenbewegungen auf den Dual-Flow-Böden durch senkrechte flächige Einbauten, sogenannte Wellenbrecher, unterbunden. Die Wellenbrecher sind anwendungstechnisch zweckmäßig in ihrer großtechnischen Anwendung 50 bis 300 mm, vorzugsweise 150 bis 200 mm hoch, sowie 500 bis 6000 mm, vorzugsweise 1000 bis 3000 mm lang (ihre Länge kann dem Bodendurchmesser oder einem Teil des Bodendurchmessers gleich sein). Bevorzugt sitzen sie mit ihrer Unterkante nicht unmittelbar auf der Oberseite des Dual-Flow-Bodens auf, sondern sind mittels kleiner Füßchen bzw. Stege auf dem Dual-Flow-Boden so aufgesetzt, dass der Abstand ihrer Unterkannte zu Oberseite des Regensiebbodens 10 bis 60 mm, vorzugsweise 30 bis 50 mm beträgt. Die Anzahl der Stege beträgt pro Wellenbrecher 1 bis 10. Der Abstand der Wellenbrecher untereinander beträgt anwendungstechnisch zweckmäßig 100 bis 1000 mm, häufig 150 bis 500 mm. Die Flächensegmente zwischen zwei Wellenbrechern betragen normalerweise ≥ 0,2 m², jedoch meist ≤ 5 m², was die Anzahl der Wellenbrecher je Regensiebboden eingrenzt.

Vorstehende Maßnahmen eignen sich insbesondere für eine bevorzugte Ausführungsvariante der Dual-Flow-Böden von Beispiel und Vergleichsbeispiel in den Schriften DE-A 10243625 und DE-A 10247240.

Eine Erhöhung des Mitrissanteils in einer erfindungsgemäß zu betreibenden Trennkolonne ist z.B. in einfacher Weise dadurch möglich, dass ein Teil der Durchtrittsöffnungen der Siebböden bei gleichbleibender Last abgedeckt wird.

Selbstredend kann das erfindungsgemäße Verfahren auch in Kombination mit einzelnen oder allen in den Schriften DE-A 2027655, EP-A 937488; EP-A 1044957 und EP-A 1029573 genannten, eine unerwünschte Polymerisation mindernden, Maßnahmen angewendet werden.

Ganz generell kann das erfindungsgemäße Verfahren unter Normaldruck, Überdruck oder unter reduziertem Druck durchgeführt werden.

Im besonderen eignet sich das erfindungsgemäße Verfahren für die in der DE-A 19924532, DE-A 10243625 und DE-A 10247240 beschriebenen fraktionierenden Kondensationen von Acrylsäure enthaltenden Produktgasgemischen von heterogen katalysierten Gasphasen-Partialoxidationen von C₃-Vorläufen der Acrylsäure mit molekularem Sauerstoff in Trennkolonnen, die von unten nach oben zunächst Dual-Flow-Böden und im Anschluss daran hydraulisch abgedichtete Querstrom-Stoffaustauschböden enthalten.

Das erfindungsgemäße Verfahren ist durch eine verminderte Neigung unerwünschter Poymerisatbildung bei gleichzeitig erhöhtem Durchsatz (kg Produkt pro Stunde) charakterisiert.

Selbstredend kann auch beim erfindungsgemäßen Verfahren bei zu großen Gasbelastungsfaktoren bzw. Flüssigkeitsgeschwindigkeiten die Flüssigkeit von den Siebböden nicht mehr ausreichend abfließen, können die Dual-Flow-Böden fluten. Jenseits der Flutungsgrenze ist kein sinnvoller Kolonnenbetrieb mehr möglich.

In der Regel schließen beim erfindungsgemäßen Verfahren die verwendeten Siebböden, insbesondere die verwendeten Regensiebböden, bündig mit den Kolonnenwänden ab. Es gibt aber auch Ausführungsvarianten, bei denen zwischen Kolonnenwand und Boden ein Zwischenraum besteht, der nur teilweise durch Brücken unterbrochen ist. Neben den eigentlichen Durchtrittsöffnungen weisen beim erfindungsgemäßen Verfahren eingesetzte Regensiebböden neben den eigentlichen Durchtrittsöffnungen allenfalls noch Öffnungen auf, die z.B. eine Befestigung des Bodens auf Auflageringen oder ähnliches ermöglichen (vgl. z.B. DE-A 10159823).

Das erfindungsgemäße Verfahren eignet sich insbesondere auch für die in der DE-A 10230219 beispielhaft beschriebene Rektifikation sowie für die in der EP-A 925272 in der Stufe (b) beschriebene Absorption.

### Beispiele und Vergleichsbeispiel

### a) Vergleichsbeispiel

Eine Trennkolonne gemäß Beispiel 1 der DE-A 10247240 (54,3 m Höhe; Innendurchmesser im Bereich der Thormann-Böden 6,5 m, ansonsten 6,0 m) enthielt von unten nach oben zunächst 15 Dual-Flow-Böden (Lochdurchmesser einheitlich 14 mm, Lochanzahl einheitlich 33678, Öffnungsverhältnis einheitlich 18 %, äquidistanter Bodenabstand 380 mm, strenge Dreiecksteilung der Mittelpunkte der Durchtrittskreise, Stanzgrat der Durchtrittslöcher nach unten zeigend, nächstliegender Abstand zweier Durchtrittslochmittelpunkte 30 mm), die mit einem ersten Fangboden abgeschlossen werden; 2,9 m oberhalb dieses Fangbodens 21 weitere Dual-Flow-Böden der beschriebenen Art, Lochanzahl jedoch einheitlich 32020 und Öffnungsverhältnis einheitlich 17,4 %; 1,50 m oberhalb des letzten Dual-Flow-Bodens Beginn einer äquidistanten (Bodenabstand = 500 mm) Anordnung von 28 konventionellen einflutigen Thormann-Böden (mit in Querstrom-Richtung aufeinanderfolgenden Rinnen jeweils zueinander entgegengesetzter Strömungsrichtung, Öffnungsverhältnis 14 %, Verhältnis von Kaminfläche zu Schlitzaustrittsfläche 0,8, Kaminhöhe und Höhe des Ablaufrohres 40 mm, Bodenfreiheit der Glocken 10 mm, Schlitzhöhe 15 mm, der Winkel zwischen ausgestelltem Schlitz und Längskante der Haube = 30 Grad, Länge der Längskante der Haube maxi-mal 800 mm, im Randbereich der Kolonne Verringerung der Haubenlänge auf bis zu 200 mm, Abstand zwischen zwei in Querstromrichtung auf einer Linie befindlichen Hauben 66 mm, Ablauffläche des Ablaufschachtes 1,5 % bezogen auf die Querschnittsfläche des Bodens, Breite zwischen den beiden unteren Längsrändern einer Haube 64 mm); 1,70 m oberhalb des obersten Thormann-Bodens befindet sich ein weiterer, zweiter, Sammelboden; 2300 mm oberhalb dieses Sammelbodens sind in äquidistanter Anordnung (Bodenabstand = 500 mm) 11 zweiflutige Ventilböden (Höhe des Ablaufrohres 35 mm, Öffnungsverhältnis 18 %, die Summe der Ablaufflächen der Ablaufschächte von zwei aufeinanderfolgenden Ventilböden 10 % der Kolonnenquerschnittsfläche).

Die Trennkolonne wurde wie in der DE-A 10247240 beschrieben betrieben. Das ihr zugeführte, auf T = 132°C gekühlte, Gasgemisch einer Partialoxidation enthielt folgende Gehalte:
26 Gew.-% Acrylsäure
0,3 Gew.-% Essigsäure,
4,3 Gew.-% Wasser,
0,03 Gew.-%Ameisensäure,
0,07 Gew.-%Formaldehyd,
0,08 Gew.-%Acrolein,
0,02 Gew.-%Propionsäure,
0,4 Gew.-% Furfurale,
0,003 Gew.-%Allylacrylat,
0,6 Gew.-% Benzaldehyd,
6,7 Gew.-% Maleinsäureanhydrid,
0,02 Gew.-%Benzoesäure,
0,09 Gew.-%Acryloylpropionsäure,
1,6 Gew.-% Kohlendioxid,
0,5 Gew.-% Kohlenmonoxid,
0,5 Gew.-% Propan,
0,2 Gew.-% Propen,
2,8 Gew.-% Sauerstoff und
55,7 Gew.-%Stickstoff.

Wie in der DE-A 10247240 beschrieben, wurde dieses Gasgemisch in der Trennkolonne aufgetrennt in eine 97,1 gew.-%ige Acrylsäure (Abzug vom ersten Fangboden bzw. Sammelboden), einen Abgasstrom mit 0,1 Gew.-% Acrylsäure (Austritt am Kopf der Trennkolonne), Sauerwasser mit 5,5 Gew.-% Acrylsäure (Abzug vom zweiten Fangboden bzw. Sammelboden) und in ein Schwersiedergemisch mit 22,8 Gew.-% Acrylsäure.

Die Temperatur am Kolonnenkopf betrug 36 °C, der Druck am Kolonnenkopf betrug 1,2 bar, das Rücklaufverhältnis lag bei 4,3. Die Sumpftemperatur betrug 132 °C und der Druck unmittelbar über der Sumpfoberfläche lag bei 1,56 bar.

Die Polymerisationsinhibierung der in der Trennkolonne absteigenden Flüssigkeit erfolgte wie in der DE-A 10247240 beschrieben.

Die 21 Dual-Flow-Böden oberhalb des Abzugs der 97,1 gew.-%igen Acrylsäure wurden wie folgt betrieben:
Gasbelastung:2,1 pa^{0,5};
Flüssigkeitsbelastung: 4,5 bis 5,5 m/h;
Druckverlust: 1,6 bis 1,7 mbar/Boden;
Mitrissanteil: 10 Gew.-%.

Nach einer Laufzeit von 35 Tagen wies die Abfolge der 21 Dual-Flow-Böden insgesamt etwa 50 kg unerwünschtes Polymerisat auf.

Bemerkung: Die Polymerisationsinhibierung kann auch wie in der DE-A 10200583 beschrieben erfolgen.

### b) Beispiel 1

Es wurde wie im Vergleichsbeispiel verfahren. Im Bereich der 21 Dual-Flow-Böden wurde jedoch auf jedem Dual-Flow-Boden bei Aufrechterhaltung der Gasbelastung eine identische Teilmenge der Durchtrittsöffnungen abgedeckt.

Dadurch stieg der Druckverlust auf 2,4 bis 2,5 mbar/Boden.
Der Mitrissanteil je Regensiebboden stieg auf 25 Gew.-%.

Nach einer Laufzeit von 55 Tagen war der Bereich der 21 Dual-Flow-Böden immer noch frei von sichtbarer Polymerisatbildung.

Die Trennwirkung war gegenüber dem Vergleichsbeispiel im wesentlichen unverändert.

Bemerkung: Die Polymerisationsinhibierung kann auch wie in der DE-A 10200583 beschrieben erfolgen.

### c) Beispiel 2

In einer Trennkolonne, die als trennwirksame Einbauten ausschließlich 30 einheitliche Dual-Flow-Böden in äquidistanter Anordnung enthielt (Durchmesser der Böden 2300 mm, Bodenabstand 330 mm, einheitlicher Lochdurchmesser 12 mm, Öffnungsverhältnis 24 %) wurde ein n-Butylacrylat enthaltendes Stoffgemisch der nachfolgenden Gehalte rektifikativ aufgetrennt:
93,18 Gew.-%n-Butylacrylat,
5,3 Gew.-% Butoxipropionsäurebutylester,
1,3 Gew.-% Acryloylpropionsäurebutylester,
0,02 Gew.-%Butylacetat,
0,02 Gew.-%Dibutylether,
0,03 Gew.-%Propionsäurebutylester,
0,02 Gew.-%Propionsäure und
0,13 Gew.-%Phenothiazin.

Das Stoffgemisch wurde der Trennkolonne unterhalb des untersten Bodens zugeführt. Die Auftrennung erfolgte in ein Kopfprodukt, das 99,8 Gew.-% n-Butylacrylat enthielt, und in ein Schwersiedergemisch, das 27,9 Gew.-% n-Butylacrylat enthielt. Die Temperatur am Kopf der Kolonne betrug 81°C, der Kopfdruck lag bei 110 mbar und das Rücklaufverhältnis betrug 0,4. Die Temperatur im Sumpf der Kolonne betrug 122 °C und der Druck auf der Oberfläche der Sumpfflüssigkeit lag bei 185 mbar.

Die Gasbelastung betrug 2,1 Pa^{0,5}. Die Flüssigkeitsbelastung betrug 2,1 m/h.
Der Druckverlust betrug 2,5 mbar/Boden.
Der Mitrissanteil betrug 56 Gew.-%.

Nach einer Laufzeit von 155 Tagen war der Bereich der Dual-Flow-Böden frei von sichtbarem Polymerisat.

## Patentansprüche

1. Thermisches Trennverfahren zwischen wenigstens einem gasförmigen und wenigstens einem flüssigen Stoffstrom, von denen wenigstens einer Acrolein, Acrylsäure, Ester der Acrylsäure, Methacrolein, Methacrylsäure und/oder Ester der Methacrylsäure enthält, in einer trennwirksame Einbauten enthaltenden Trennkolonne, wobei es sich wenigstens bei einem Teil der trennwirksamen Einbauten um eine Abfolge von Siebböden handelt, **dadurch gekennzeichnet, dass** die Stoffströme so gewählt werden, dass dabei wenigstens ein Teil der Siebböden oberhalb eines Mitrissanteils von 10 Gew-% betrieben wird.

2. Thermisches Trennverfahren nach Anspruch 1, **dadurch gekennzeichnet, dass** die Trennkolonne als trennwirksame Einbauten ausschließlich Stoffaustauschböden enthält.

3. Thermisches Trennverfahren nach Anspruch 1 oder 2, **dadurch gekennzeichnet, dass** die Trennkolonne als trennwirksame Einbauten von unten nach oben Regensiebböden, hydraulisch abgedichtete Querstromböden und Ventilböden enthält.

4. Thermisches Trennverfahren nach Anspruch 1 oder 2, **dadurch gekennzeichnet, dass** die Trennkolonne als trennwirksame Einbauten ausschließlich Regensiebböden enthält.

5. Thermisches Trennverfahren nach einem der Ansprüche 1 bis 4, **dadurch gekennzeichnet, dass** es ein Verfahren der fraktionierenden Kondensation, der Rektifikation oder der Absorption ist.

6. Thermisches Trennverfahren nach einem der Ansprüche 1 bis 5, **dadurch gekennzeichnet, dass** wenigstens ein Teil der Siebböden bei einem Mitrissanteil von 11 bis 70 Gew.-% betrieben wird.

7. Thermisches Trennverfahren nach einem der Ansprüche 1 bis 6, **dadurch gekennzeichnet, dass** wenigstens ein Teil der Siebböden bei einem Mitrissanteil von 11 bis 30 Gew.-% betrieben wird.

8. Thermisches Trennverfahren nach einem der Ansprüche 1 bis 6, **dadurch gekennzeichnet, dass** alle Siebböden bei einem Mitrissanteil von 11 bis 70 Gew.-% betrieben werden.

9. Thermisches Trennverfahren nach einem der Ansprüche 1 bis 7, **dadurch gekennzeichnet, dass** alle Siebböden bei einem Mitrissanteil von 11 bis 30 Gew.-% betrieben werden.

10. Thermisches Trennverfahren nach einem der Ansprüche 1 bis 9, **dadurch gekennzeichnet, dass** der flüssige Stoffstrom Polymerisationsinhibitoren enthält.

11. Thermisches Trennverfahren nach einem der Ansprüche 1 bis 10, **dadurch gekennzeichnet, dass** es ein Verfahren der fraktionierenden Kondensation des Produktgasgemisches einer katalytischen Gasphasenoxidation von C3-Vorläuferverbindungen der Acrylsäure zur Herstellung von Acrylsäure ist.

## Claims

1. A thermal separating process between at least one gaseous and at least one liquid stream, of which at least one comprises acrolein, acrylic acid, esters of acrylic acid, methacrolein, methacrylic acid and/or esters of methacrylic acid, in a separating column containing separating internals, at least some of the separating internals being a sequence of sieve trays, which comprises selecting the streams in such a way that at least some of the sieve trays are operated above an entrainment fraction of 10% by weight.

2. The thermal separating process according to claim 1, wherein the separating internals contained in the separating column are exclusively mass transfer trays.

3. The thermal separating process according to claim 1 or 2, wherein the separating internals contained in the separating column are, from bottom to top, dual-flow trays, hydraulically sealed crossflow trays and valve trays.

4. The thermal separating process according to claim 1 or 2, wherein the separating internals contained in the separating column are exclusively dual-flow trays.

5. The thermal separating process according to any of claims 1 to 4, which is a process for fractional condensation, for rectification or for absorption.

6. The thermal separating process according to any of claims 1 to 5, wherein at least some of the sieve trays are operated at an entrainment fraction of from 11 to 70% by weight.

7. The thermal separating process according to any of claims 1 to 6, wherein at least some of the sieve trays are operated at an entrainment fraction of from 11 to 30% by weight.

8. The thermal separating process according to any of claims 1 to 6, wherein all of the sieve trays are operated at an entrainment fraction of from 11 to 70% by weight.

9. The thermal separating process according to any of claims 1 to 7, wherein all of the sieve trays are operated at an entrainment fraction of from 11 to 30% by weight.

10. The thermal separating process according to any of claims 1 to 9, wherein the liquid stream comprises polymerization inhibitors.

11. The thermal separating process according to any of claims 1 to 10, which is a process for fractionally condensing the product gas mixture of a catalytic gas phase oxidation of C3 precursor compounds to acrylic acid for preparing acrylic acid.

## Revendications

1. Procédé de séparation thermique entre au moins un flux de matière gazeux et au moins un flux de matière liquide, dont au moins l'un contient de l'acroléine, de l'acide acrylique, des esters de l'acide acrylique, de la méthacroléine, de l'acide méthacrylique et/ou des esters de l'acide méthacrylique, dans une colonne de séparation contenant des encastrements actifs en séparation, au moins une partie des encastrements actifs en séparation étant une succession de plateaux perforés, **caractérisé en ce que** les flux de matière sont choisis de maniéré telle qu'au moins une partie des plateaux perforés est exploitée au-dessus d'une proportion d'entraînement de 10% en poids.

2. Procédé de séparation thermique selon la revendication 1, **caractérisé en ce que** la colonne de séparation contient, comme encastrement actifs en séparation, exclusivement des plateaux d'échange de matière.

3. Procédé de séparation thermique selon la revendication 1 ou 2, **caractérisé en ce que** la colonne de séparation contient, comme encastrements actifs en séparation, de bas en haut, des plateaux à double flux, des plateaux à flux transversal hydrauliquement étanches et des plateaux à soupape.

4. Procédé de séparation thermique selon la revendication 1 ou 2, **caractérisé en ce que** la colonne de séparation contient, comme encastrements actifs en séparation, exclusivement des plateaux à double flux.

5. Procédé de séparation thermique selon l'une quelconque des revendications 1 à 4, **caractérisé en ce qu'**il s'agit d'un procédé de condensation fractionnée, de rectification ou d'absorption.

6. Procédé de séparation thermique selon l'une quelconque des revendications 1 à 5, **caractérisé en ce qu'**au moins une partie des plateaux perforés est exploitée à une proportion d'entraînement de 11 à 70% en poids.

7. Procédé de séparation thermique selon l'une quelconque des revendications 1 à 6, **caractérisé en ce qu'**au moins une partie des plateaux perforés est exploitée à une proportion d'entraînement de 11 à 30% en poids.

8. Procédé de séparation thermique selon l'une quelconque des revendications 1 à 6, **caractérisé en ce que** tous les plateaux perforés sont exploités à une proportion d'entraînement de 11 à 70% en poids.

9. Procédé de séparation thermique selon l'une quelconque des revendications 1 à 7, **caractérisé en ce que** tous les plateaux perforés sont exploités à une proportion d'entraînement de 11 à 30% en poids.

10. Procédé de séparation thermique selon l'une quelconque des revendications 1 à 9, **caractérisé en ce que** le flux de matière liquide contient des inhibiteurs de polymérisation.

11. Procédé de réparation thermique selon l'une quelconque des revendications 1 à 10, **caractérisé en ce qu'**il s'agit d'un procédé de condensation fractionnée du mélange gazeux de produits d'une oxydation catalytique en phase gazeuse de composés précurseurs en c3 de l'acide acrylique pour la préparation d'acide acrylique.
